Europäisches Patentamt

European Patent Office

Office européen des brevets

(11) Veröffentlichungsnummer: **0 388 974**
**A2**

(12) **EUROPÄISCHE PATENTANMELDUNG**

(21) Anmeldenummer: 90105553.3

(22) Anmeldetag: 23.03.90

(51) Int. Cl.⁵: **G01N 33/564, G01N 33/74,**
**G01N 30/00**

(30) Priorität: 23.03.89 DE 3909690

(43) Veröffentlichungstag der Anmeldung:
26.09.90 Patentblatt 90/39

(84) Benannte Vertragsstaaten:
AT BE CH DE DK ES FR GB GR IT LI LU NL SE

(71) Anmelder: **Henning Berlin GmbH Chemie-**
**und Pharmawerk**
**Komturstrasse 58-62**
**D-1000 Berlin 42(DE)**

(72) Erfinder: **Kolb-Bachofen, Victoria, Dr.**
**Konrad-Hagius-Strasse 7**
**D-4000 Düsseldorf 13(DE)**

(74) Vertreter: **Andrae, Steffen, Dr. et al**
**Steinstrasse 44**
**D-8000 München 80(DE)**

(54) Verfahren zur Früherkennung von Änderungen des Immunstatus des Menschen.

(57)     2.1. Die bisherigen Verfahren zur Bestimmung und Früherkennung von Änderungen des Immunstatus des Menschen beruhen in erster Linie auf einer Interpretation des Blutbilds und sind nicht in der Lage, frühe Änderungen des Immunstatus anzuzeigen.

2.2. Indem man in einer Vollblut-, Plasma- oder Serumsprobe eines menschlichen Patienten nebeneinander den Gehalt an mindestens einem Thymosin der α-Reihe als Maß für die Immunstimmulation als auch den Gehalt an mindestens einem Thymosin der β-Reihe als Maß für die Immunsuppression bestimmt, ist eine neuartige Bestimmung des Immunstatus des Menschen sowie die Früherkennung von Änderungen dieses Immunstatus möglich.

2.3. Diagnostisches Verfahren, insbesondere zur Vorhersage von Änderungen des klinischen Bildes und der Überprüfung der Therapie bei Autoimmunerkrankungen oder anderen Entzündungsprozessen.

EP 0 388 974 A2

## Verfahren zur Früherkennung von Änderungen des Immunstatus des Menschen

Die Erfindung betrifft Verfahren zur Bestimmung und Früherkennung von Änderungen des Immunstatus des Menschen, insbesondere zur Früherkennung von Rezidiv oder Remission einer Autoimmunerkrankung.

Die Gewinnung von Daten, die sich mit dem Immunstatus eines Patienten korrelieren lassen, ist bei zahlreichen Erkrankungen als Therapievoraussetzung oder als eine die Therapie begleitende Maßnahme von großer Wichtigkeit. In der gegenwärtigen klinischen Praxis gewinnt man derartige Daten dabei in erster Linie aus dem Blutbild. Ein derartiges Verfahren ist jedoch nur beschränkt aussagekräftig und liefert eher ein Bild eines aufgrund einer Änderung des Immunstatus bereits erreichten klinischen Zustands, als daß es eine einsetzende Änderung dieses Zustands signalisiert.

Andere Diagnoseverfahren liefern nur Teilantworten, indem sie auf ganz spezielle Fragestellungen zugeschnitten sind, wie beispielsweise die IgE-Bestimmung im Rahmen einer Diagnose bei allergischen Symptomen. Andere Untersuchungsmethoden, z. B. histologische Untersuchungen, sind für diagnostische Zwecke nur begrenzt durchführbar, belasten den Patienten und sind kaum als Routineuntersuchungen durchführbar.

Verfahren, die eine Früherkennung von Änderungen des Immunstatus ermöglichen, sind in der klinischen Praxis derzeit unbekannt. Das in den letzten Jahren erheblich angewachsene Wissen über die Feinsteuerung der Immunantwort, die als Produkt der gegenläufigen Reaktionen des Immunsystems, Immunstimulation und Immunsuppression,beschreibbar ist, hat die Interpretation der erhältlichen Daten eher erschwert als erleichtert.

Besondere Bedeutung kommt der Ermittlung des Immunstatus dabei naturgemäß im Falle solcher Erkrankungen zu, die ihre Ursache direkt im Immunsystem selbst haben, wozu insbesondere die Auto immunerkrankungen gehören. Zu diesen Erkrankungen zählen zahlreiche schwere Erkrankungen wie der systemische Lupus erythematodes, die Muskelschwäche (Myasthenia gravis), Schilddrüsenentzündung, rheumatische Gelenkentzündungen, einige Formen fortschreitender Lebererkrankungen sowie insbesondere auch der Diabetes Typ I. Bei derartigen Erkrankungen lassen sich Änderungen des Immunstatus meist direkt mit Remission oder Rezidiv der Krankheit korrelieren.

· Die Früherkennung einer Änderung des Immunstatus ist dabei wichtig, um die zu erwartende Änderung des klinischen Bildes rechtzeitig vorherzusagen und um möglichst frühe Informationen darüber zu erhalten, ob therapeutische Interventionen die gewünschte Wirksamkeit entwickeln, oder umgekehrt, ob eine klinische Verschlechterung (Krankheitsschub) droht.

Die Früherkennung einer Änderung des Immunstatus ist für den frühen Nachweis einer normalen Immunreaktion (Abwehrlage) bei Infektionen bzw. dem frühen Nachweis von Immundefekten generell wichtig, erleichtert den Nachweis eines chronischen Entzündungsgeschehens und ist ganz besonders wichtig, um Rezidiv oder Remission einer Autoimmunkrankheit möglichst früh zu erkennen. Selbstverständlich ist die Früherkennung einer Änderung des Immunstatus auch im Zusammenhang mit anderen Entzündungsprozessen einschließlich Transplantatabstoßungsreaktionen und Tumorabwehr wichtig.

Als therapiebegleitende Untersuchung könnte durch eine Früherkennung einer Änderung des Immunstatus der Effekt einer immunmodulatorischen Therapie (Stimulation, Suppression) früh festgestellt werden, so daß eine erfolglose oder gar unerwünschte Wirksamkeit entfaltende Therapie abgebrochen oder geändert werden kann, ohne daß sich Erfolg oder Mißerfolg der Therapie am klinischen Zustand des Patienten zeigen müssen.

Es ist Aufgabe der vorliegenden Erfindung, Verfahren zur Bestimmung und zur Früherkennung einer Änderung des Immunstatus zu schaffen, die als typische Laborverfahren unter Verwendung üblicher Blutproben durchgeführt werden können.

Diese Aufgabe wird durch ein Verfahren gemäß Patentanspruch 1 bzw. 6 gelöst.

Vorteilhafte Ausgestaltungen des Verfahrens zur Früherkennung von Änderungen des Immunstatus sind den Unteransprüchen 2 bis 5 zu entnehmen.

Die vorliegende Erfindung beruht auf der Erkenntnis, daß der Gehalt an Thymosin $\alpha$ in einer klinischen Probe ein Maß für die Immunstimulation darstellt, und daß der entsprechende Gehalt an Thymosin $\beta$ ein Maß für die Immunsuppression ist. Die einfache Bestimmung der absoluten Spiegel von Thymosinen der beiden Reihen, insbesondere von Thymosin $\alpha_1$ und Thymosin $\beta_4$, kann somit bereits eine Aussage über den Immunstatus eines Patienten liefern. Damit die durch einfache Bestimmung der absoluten Spiegel der beiden Thymosine erhaltenen Daten für diagnostische Zwecke brauchbar sind, müßten einen Normzustand (Gesundheit) anzeigende Normbereiche existieren oder mit ausreichender statistischer Sicherheit noch festgelegt werden. Es erscheint wahrscheinlich, daß das mit der Zunahme der verfügbaren experimentellen Daten der Fall sein wird. Die gleichzeitige Bestimmung der absoluten Spiegel von Thymosin $\alpha$, insbesondere $\alpha_1$, und Thymosin $\beta$, insbesondere Thy-

mosin $\beta_4$, könnte dann einen Sonderfall eines Verfahrens zur Bestimmung des Immunstatus gemäß der vorliegenden Erfindung darstellen, die auf der obigen neuen Erkenntnis beruht.

Die Ausgestaltung als Früherkennungs-Verfahren erscheint jedoch wichtiger und erfolgversprechender. Bei einer solchen Ausgestaltung des erfindungsgemäßen Verfahrens wird die Veränderung des Immunstatus anhand der Thymosin $\alpha$- und $\beta$-Gehalte ermittelt, wobei von einem individuell bestimmten Ausgangszustand ausgegangen werden kann.

Die bei dem erfindungsgemäßen Verfahren nebeneinander bestimmten Thymosine der $\alpha$-Reihe und $\beta$-Reihe sind seit einer Reihe von Jahren bekannt. Sie gehören zur Familie der Thymosine, die hormonartige Peptide sind, die ursprünglich aus der Thymusdrüse isoliert wurden. Die Thymosine werden aufgrund ihres Wanderungsverhaltens im elektrischen Feld bei der isoelektrischen Fokussierung (Polyacrylamidgel) in $\alpha$-, $\beta$- und $\gamma$-Thymosine unterteilt. Eine zusammenfassende Übersicht über die chemische Natur und die bisher bekannten biologischen Aktivitäten der verschiedenen Thymosine findet sich z. B. in "Biology and Chemistry of Thymosin Peptides", von B. L. Spangelo, N. R. Hall und A. L. Goldstein in : Annals New York Academy of Sciences, 1986, Seiten 196 bis 204; in:"Thymosins: hormones of the thymus gland" von Karen K. Oates and Allan L. Goldstein in: Trends in Pharmacological Sciences, Vol. 5, Nr. 8, Seiten 347 bis 352, 1984 oder in:"Thymosins, Lymphokines and the Immunology of Aging" von Marion M. Zatz, Allan L. Goldstein in: Gerontology 31: 263 bis 277 (1985). Der Gehalt der am besten untersuchten Thymosine $\alpha_1$ und $\beta_4$ in menschlichem Serum wird als in den Bereichen von 500 bis 1000 pg/ml bzw. 300 bis 1000 ng/ml liegend angegeben.

Die Gehalte der Thymosine, insbesondere der Thymosine $\alpha_1$ und $\beta_4$, in einer Probe können nach verschiedenen an sich bekannten Verfahren bestimmt werden. Ein Verfahren zur Bestimmung von Thymosin $\beta_4$ durch Hochdruck-Flüssigkeitschromatographie (HPLC) ist beschrieben in Analytical Biochemistry 156, 390-396 (1986) sowie erwähnt in Journal of Biological Response Modifiers, 7:91-96, 1988. Immunchemische Bestimmungsverfahren, nämlich Immunoassays, für die Thymosine $\alpha_1$ und $\beta_4$ sind ebenfalls beschrieben. So werden im Journal of Immunological Methods, 81 (1985), 199 bis 205 bzw. ibid.89 (1986) 9 bis 17, Radioimmunoassays für Thymosin $\alpha_1$ beschrieben. Im Journal of Immunological Methods, 60 (1983), 53 bis 60, wird die Bestimmung von Thymosin $\alpha_1$ mit Hilfe eines Dissoziations-MikroELISA beschrieben. Ein Radioimmunoassay für Thymosin $\beta_4$ ist beschrieben in Immunopharmacology, 7 (1984) 9 bis 16, und ein Enzymimmunoassay (EIA) für Thymosin $\beta_4$ in Journal of Biological Response Modifiers, 7:91 bis 96 (1988).

Diese Bestimmungsverfahren wurden für wissenschaftliche Untersuchungszwecke entwickelt und habennoch keine Bedeutung für die klinische Diagnostik erlangt.

Einer der Gründe dafür ist, daß bisher zwar ein wachsendes Wissen zur biologischen Wirksamkeit der Thymosine und auch der einzelnen Thymosintypen angesammelt worden ist, daß jedoch eine Korrelation der $\alpha$- und $\beta$-Thymosinspiegel zu bestimmten physiologischen Zuständen oder Krankheitsbildern noch nicht hergestellt wurde. Die Messung der $\alpha$- und $\beta$-Thymosingehalte für diagnostische Zwecke wurde daher noch nicht durchgeführt.

Im Rahmen eines Forschungsprojekts, das den Diabetes Typ I betraf und bei dem über längere Zeiträume gleichzeitig die Gehalte von Thymosin $\alpha_1$ und Thymosin $\beta_4$ von Patienten gemessen wurden, wurde nunmehr überraschend festgestellt, daß sich die gemessenen Thymosingehalte mit dem klinischen Verlauf korrelieren lassen, und zwar dann, wenn man den Wert für Thymosin $\alpha$ bzw. insbesondere Thymosin $\alpha_1$ als Maß für die Immunstimulation nimmt und den Gehalt an Thymosin $\beta$, insbesondere Thymosin $\beta_4$, als Maß für die Immunsuppression. Die Kombination der für die absoluten Spiegel der beiden Substanzen erhaltenen Werte liefert dabei eine Aussage, die aufgrund der gleichzeitigen Berücksichtigung der Immunstimulation und der Immunsuppression eine gute Aussage zum Immunstatus zum Zeitpunkt der Probennahme liefert.

Bei den Untersuchungen stellte sich darüber hinaus jedoch auch noch heraus, daß die Änderungen der Blutspiegel der Thymosine $\alpha$ und $\beta$ frühzeitig Veränderungen des Immunstatus anzeigen. So bedeutet ein Anstieg der Werte für Thymosin $\alpha$ eine verstärkte Immunstimulation, ein Anstieg der Werte für Thymosin $\beta$ eine verstärkte Immunsuppression. Umgekehrt gilt, daß ein Abfall der Werte für Thymosin $\alpha$ eine verminderte Immunstimulation und ein Abfall der Werte für Thymosin $\beta$ eine verminderte Immunsuppression anzeigt. Durch die gleichzeitige Berücksichtigung der beiden Werte kann ein gutes Bild des Immunstatus und seiner Veränderungen erhalten werden, und,wie bereits eingangs angedeutet, können darauffolgende Änderungen des klinischen Bildes frühzeitig vorhergesagt werden und es kann die Wirksamkeit von therapeutischen Inter ventionen früh beurteilt werden.

Bei einer Untersuchung von 34 Seren von Patienten mit Morbus Basedow, einer Schilddrüsen-Autoimmunerkrankung, konnte festgestellt werden, daß bei diesen Patienten die Thymosin $\beta_4$-Spiegel gegenüber Normalseren signifikant erniedrigt waren. Die Thymosin $\alpha_1$-Spiegel zeigten keine signifi-

kanten Anormalitäten. Auch in diesem Fall ist somit eine diagnostisch relevante Korrelation zwischen Immunstatus und dem Gehalt an Thymosin in der $\alpha$- und der $\beta$-Reihe möglich, und die Bildung eines Quotienten der für die beiden Thymosin-Typen gemessenen Spiegel erfaßt auch solche Fälle, bei denen sich die Änderungen des Immunstatus vor allem als Änderung des Spiegels eines der beiden Thymosin-Typen auswirken.

Durch die vorliegende Erfindung wird somit erstmals die Bestimmung von Thymosinen der $\alpha$-Reihe neben Thymosinen der $\beta$-Reihe für einen neuen diagnostischen Zweck beschrieben, nämlich für die Bestimmung des Immunstatus bzw. die Früherkennung von Änderungen des Immunstatus.

in einer Vollblut-, Plasma- oder Serumsprobe eines menschlichen Patienten sowohl den Gehalt an mindestens einem Thymosin der $\alpha$-Reihe als auch dem Gehalt an mindestens einem Thymosin der $\beta$-Reihe bestimmt und

den Immunstatus dadurch ermittelt, daß man entweder die absoluten Spiegel für $\alpha$-Thymosin mit der Immunstimulation und $\beta$-Thymosin mit der Immunsuppression korreliert und mit den Werten für einen Normalzustand vergleicht, oder daß man einen Quotienten aus den gemessenen Spiegeln für $\alpha$-Thymosin und $\beta$-Thymosin bildet und diesen Quotienten mit dem entsprechenden Quotienten für einen Normalzustand vergleicht.

## Ansprüche

1. Verfahren zur Früherkennung von Änderungen des Immunstatus des Menschen, insbesondere zur Früherkennung von Rezidiv oder Remission einer Autoimmunerkrankung,
dadurch gekennzeichnet, daß man
in einer ersten Vollblut-, Plasma- oder Serumsprobe eines menschlichen Patienten sowohl den Gehalt an mindestens einem Thymosin der $\alpha$-Reihe als auch den Gehalt an mindestens einem Thymosin der $\beta$-Reihe bestimmt,
diese Bestimmungen mindestens einmal an einer nach einem klinisch signifikanten Zeitraum entnommenen weiteren Probe des gleichen Patienten wiederholt und
die Änderung des Immunstatus dadurch ermittelt, daß man die Änderung des Gehalts an $\alpha$-Thymosin mit der Änderung der Immunstimulation und die Änderung des Gehalts an $\beta$-Thymosin mit der Änderung der Immunsupression korreliert.

2. Verfahren nach Anspruch 1, dadurch gekennzeichnet, daß man die Gehalte an Thymosin $\alpha_1$ und Thymosin $\beta_4$ bestimmt.

3. Verfahren nach Anspruch 1 oder 2, dadurch gekennzeichnet, daß man den Gehalt der verschiedenen Thymosine in den Proben durch Hochdruck-Flüssigkeitschromatographie (HPLC), Gaschromatographie und/oder immunchemisch bestimmt.

4. Verfahren nach einem der Ansprüche 1 bis 3, dadurch gekennzeichnet, daß man die Bestimmungen an Proben durchführt, die in einem zeitlichen Abstand im Bereich von zwei Wochen bis zu sechs Monaten entnommen wurden.

5. Verfahren nach einem der Ansprüche 1 bis 4, dadurch gekennzeichnet, daß man es zur Früherkennung von Rezidiv oder Remission eines Diabetes Typ I durchführt.

6. Verfahren zur Bestimmung des Immunstatus des Menschen,
dadurch gekennzeichnet, daß man